# EUROPÄISCHE PATENTSCHRIFT

(11) **EP 0 542 082 B1**
(45) Veröffentlichungstag und Bekanntmachung des Hinweises auf die Patenterteilung: **25.02.1998**
(21) Anmeldenummer: 92118739.9
(22) Anmeldetag: 02.11.1992
(51) Int. Cl.: C09B 62/04, C09B 62/503, C07D 251/50, C09B 62/44

(54) **Verfahren zur kontinuierlichen Umsetzung von Difluortriazinyl-Verbindungen mit Aminen**
Process for continuously reacting difluortriazinyl compounds with amines
Méthode de réaction en continu de composés de type difluortriazine avec des amines

(30) Priorität: 13.11.1991 DE 4137292
(43) Veröffentlichungstag der Anmeldung: 19.05.1993
(73) Patentinhaber: BAYER AG, 51368 Leverkusen (DE)
(72) Erfinder: Hoppe, Manfred, Dr., W-5067 Kürten (DE); Müllers, Wolfgang, Dr., W-5060 Bergisch Gladbach 1 (DE); Frosch, Hans-Georg, Dr., W-5000 Köln 91 (DE); Sommer, Richard, Dr., W-5068 Odenthal (DE); Arnold, Siegbert, Dr., W-5300 Bonn (DE); Reddig, Wolfram, Dr., W-5060 Bergisch Gladbach 1 (DE)

(56) Entgegenhaltungen:
- EP-A- 0 053 097
- EP-A- 0 172 790
- EP-A- 0 458 110
- EP-A- 0 494 456
- FR-A- 2 367 755

## Beschreibung

Die Anmeldung betrifft ein Verfahren zur kontinuierlichen Umsetzung von Difluortriazinyl-Verbindungen mit Aminen.

Die Umsetzung von Difluortriazinyl-Verbindungen I 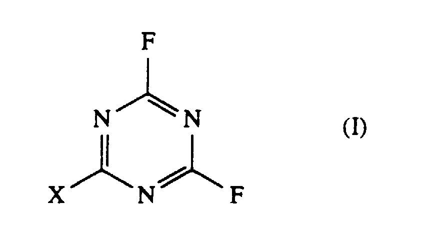 mit Aminen oder chromophorhaltigen Aminen wird nach bekannten Verfahren durch Einstellen der Temperatur und des pH-Wertes nach Zusammenfügen der Komponenten durchgeführt. Dies erfolgt üblicherweise in Rührwerksbehältern, was jedoch Nachteile mit sich bringt, weil durch die Zugabe einer der Komponenten und/oder durch Einstellung des pH-Wertes pH-Gradienten und/odcr Stöchiometrie-Gradienten entstehen.

Diese Nachteile werden hervorgerufen durch die geringe spezifische Mischenergie in Rührwerksbehältern. Bei der Umsetzung von besonders reaktionsfähigen Verbindungen wie Difluortriazinen mit Aminen führen diese Nachteile zu Ausbeuteverlusten durch Hydrolyse und Nebenreaktionen.

Gemäß DE-A- 27 46 109 erfolgt die Herstellung von monoacylierten, wasserlöslichen, organischen Aminoverbindung mit Cyanurfluorid durch kontinuierliche Umsetzung in einem Reaktor.

Aus EP-172 790 ist bekannt, Monokondensationsprodukte von Cyanurfluorid dadurch zu erhalten, daß man zunächst Cyanurfluorid und ein Amin gleichzeitig und kontinuierlich in einen ersten Reaktor leitet, indem eine intensive Durchmischung erfolgt, und die Reaktionsmischung anschließend zur Vervollständigung der Reaktion in einen zweiten Reaktor leitet, indem nur geringe Rückvermischung gewährleistet ist.

Gemäß EP-A-494 456 (dieses Dokument fällt unter Artikel 54(3) EPÜ) wird ein verbessertes Verfahren zur Herstellung von durch einen faserreaktiven Rest substituierten 2-Amino-1-sulfonaphthalins beschrieben. Die Herstellung erfolgt durch Umsetzung von der entsprechenden Aminoverbindung und einer fluorhaltigen Reaktivkomponente, wobei beide Reaktanden gleichzeitig in den Reaktor unter Reaktion eindosiert werden.

Es besteht die Aufgabe, ein verbessertes Verfahren für die Umsetzung von Triazinylverbindungen mit Aminen, insbesondere Aminonaphtholsulfonsäuren, zu finden.

Die Erfindung betrifft ein verbessertes Verfahren zur Umsetzung von Difluortriazinyl-Verbindungen der Formel I 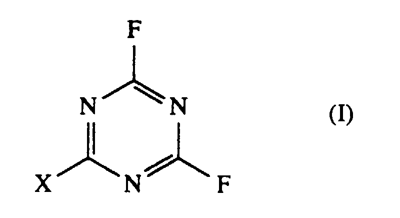 - bevorzugt sulfogruppenhaltige - mit Aminen Y-H zu Reaktivfarbstoffen oder deren Vorprodukten der Formel II 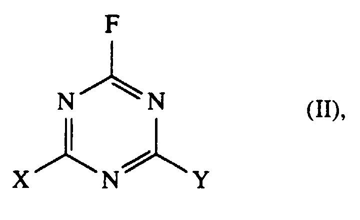 wobei:
- X-H und Y-H: unabhängig voneinander ein primäres oder sekundäres aliphatisches, aromatisches oder heterocyclisches Amin ist, das an einem oder beiden Resten des Stickstoff-Atomes substituiert sein kann, vorzugsweise durch OH, OSO₃H, SO₃H, COOH, Cl, OC₁-C₄-Alkyl, C₁-C₄-Alkyl, SO₂-(CH₂)₂-OSO₃H, SO₂-CH=CH₂, SO₂-(CH₂)₂-Cl, NH-Acyl, NH-C₁-C₄-Alkyl, CONH(CH₂)₂-O-(CH₂)₂-SO₂-CH=CH₂, O(CH₂)₂SO₂-CH=CH₂, O-Acyl,
wobei X-H und/oder Y-H auch chromophorhaltige Amine sein können, dadurch gekennzeichnet, daß man die Difluortriazinyl-Verbindung vom Typ I als wäßrige oder wäßrig-organische Lösung oder als Suspension und das Amin Y-H kontinuierlich in einem Reaktor unter Vermeidung, zumindest unter Minimierung von Rückvermischung zur Umsetzung bringt.

Geeignete aliphatische und heterocyclische Amine X-H bzw. Y-H sind beispielsweise solche, in denen
- X bzw. Y: ein Rest der Formel 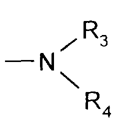 ist,
in welchen
- R₃ =: ein Wasserstoffatom oder ein gegebenenfalls substituierter niederer aliphatischer Rest oder ein cycloaliphatischer Rest ist und
- R₄=: ein Wasserstoffatom oder ein gegebenenfalls substituierter niederer aliphatischer Rest oder ein gegebenenfalls substituierter aromatischer carbocyclischer Rest oder eine niedere Alkoxygruppe oder die Cyangruppe oder die Gruppe der Formel -CS-NH₂ oder eine gegebenenfalls substituierte Aminogruppe bedeutet oder
- R₃ und R₄: zusammen mit dem Stickstoffatom ein niederes Alkylen und gegebenenfalls Heteroatome, wie beispielsweise ein Stickstoff- oder Sauerstoffatom, enthaltenden Ring bilden, wie beispielsweise einen Morpholin-, Piperidin-oder Piperazin-Ring.

Niedere aliphatische Reste sind insbesondere niedere Alkyl- und Alkenylreste.

Die in den obigen Definitionen verwendete Angabe "niedere" bedeutet hier wie im folgenden, daß der in der Gruppe enthaltene Alkyl- oder Alkylenrest aus 1 bis 4 C-Atomen und der Alkenylrest aus 2 bis 4 C-Atomen besteht.

Substituierte niedere Alkylreste sind beispielsweise Alkylgruppen von 1 bis 4 C-Atomen, die durch einen oder zwei Substituenten aus der Gruppe Acetylamino, Hydroxy, Sulfato, β-Sulfatoethylsulfonyl, O-(CH₂)₂-SO₂-CH=CH₂, β-Thiosulfatoethylsulfonyl, niederes Alkoxy, Sulfo, Carboxy, Phenyl, Naphthyl und/oder durch Sulfo, Carboxy, β-Sulfatoethylsutfonyl, β-Thiosulfatoethylsulfonyl, Methyl, Ethyl, Methoxy, Ethoxy, Chlor, Sulfamoyl oder Carbamoyl substituiertes Phenyl substituiert sind.

Geeignete aromatische Amine X-H bzw. Y-H sind beispielsweise solche der Formel IV 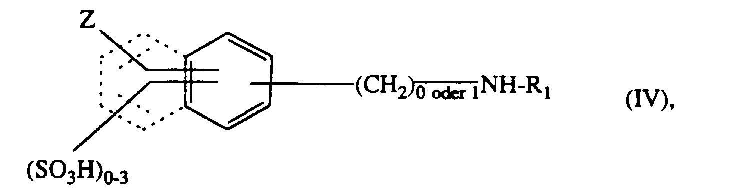 wobei:
- R₁=: H, C₁-C₄-Alkyl, das durch OR, OSO₃H, SO₃H, COOR oder Halogen substituiert sein kann,
- R =: H, CH₃, C₂H₅,
- Z =: H, R, OH, Cl, OR, NH-Acyl, NHR, SO₂-CH=CH₂, SO₂-CH₂-CH₂-OSO₃H, O-Acyl, SO₂-CH₂-CH₂-Cl oder CONH(CH₂)₂O(CH₂)₂-SO₂-CH=CH₂.

### Besonders geeignete aromatische Amine sind beispielsweise:

1-Aminobenzol-2-sulfonsäure, 1-Aminobenzol-3-sulfonsäure, 1-Aminobenzol-2-sulfonsäure, 1-Amino-4-methylbenzol-3-sulfonsäure, 1-Amino-4-methoxybenzol-3-sulfonsäure, 1-Amino-2-methylbenzol-4-sulfonsäure, 1-Amino-3-methylbenzol-4-sulfonsäure, 1-Aminobenzol-3,5-disulfonsäure, 2-Amino-5-sulfobenzoesäure, 1-Aminonaphthalin-4-sulfonsäure, 1-Aminonaphthalin-5-sulfonsäure, 1-Aminonaphthalin-6-sulfonsäure, 2-Aminonaphthalin-5-sulfonsäure, 2-Aminonaphthalin-7-sulfonsäure, 2-Aminonaphthalin-4,8-disulfonsäure, 2-Aminonaphthalin-5,7-disulfonsäure, 1,4-Diaminobenzol-2,5-disulfonsäure, 1,3-Diaminobenzol-4-sulfonsäure, 1,4-Diaminobenzol-2-sulfonsäure, 1,3-Diaminobenzol-4,6-disulfonsäure, 1-Amino-5-hydroxynaphthalin-7-sulfonsäure, 1-Amino-8-hydroxynaphthalin-4-sulfonsäure, 1-Amino-8-hydroxynaphthalin-3-sulfonsäure, 1-Amino-8-hydroxynaphthalin-5-sulfonsäure, 2-Amino-5-hydroxynaphthalin-7-sulfonsäure, 2-Amino-6-hydroxynaphthalin-8-sulfonsäure, 2-Amino-8-hydroxynaphthalin-6-sulfonsäure, 2-Methylamino-5-hydroxynaphthalin-7-sulfonsäure, 2-Ethylamino-5-hydroxynaphthalin-7-sulfonsäure, 2-Methylamino-8-hydroxynaphthalin-6-sulfonsäure, 2-Ethylamino-8-hydroxynaphthalin-6-sulfonsäure, 1-Amino-6-hydroxynaphthalin-3,8-disulfonsäure, 1-Amino-8-hydroxynaphthalin-3,6-disulfonsäure, 1-Amino-8-hydroxynaphthalin-2,4-disulfonsäure, 1-Amino-8-hydroxynaphthalin4,6-disulfonsäure, 1-Amino-8-hydroxynaphthalin-3,5-disulfonsäure, 2-Amino-5-hydroxynaphthalin-7, 1-disulfonsäure und 2-Amino-8-hydroxynaphthalin-3,6-disulfonsäure.

Anilin-4-β-sulfatoethylsulfon, Anilin-4-β-thiosulfatoethylsulfon, Anilin-3-β-sulfatoethylsulfon, 2-Methoxyanilin-5-β-sulfatoethylsulfon, 2-Methoxyanilin-5-β-thiosulfatoethylsulfon, 4-Methoxyanilin-3-β-sulfatoethylsulfon, 2,5-Dimethoxyanilin-4-β-sulfatoethylsulfon, 2,5-Dimethoxyanilin-4-β-sulfatoethylsulfon, 2-Methoxy-5-methylanilin-4-β-sulfatoethylsulfon, Anilin-2-β-sulfatoethylsulfon, 2-Chloranilin-5-β-sulfatoethylsulfon, 4-Chloranilin-3-β-sulfatoethylsulfon, 3-(3- oder 4-Aminobenzoyl)-aminophenyl-β-sulfatoethylsulfon, 6-Carboxyanilin-3-vinylsulfon, 2-Sulfoanilin-4-β-sulfatoethylsulfon, 2-Sulfoanilin-4-vinylsulfon, 2,4-Disulfoanilin-5-vinylsulfon, 2-Hydroxyanilin-5-β-sulfatoethylsulfon, 2-Hydroxyanilin-4-β-sulfatoethylsulfon, 3-Sulfo-2-hydroxyanilin-5-β-sulfatoethylsulfon, 2-Naphthylamin-8-β-sulfatoethylsulfon, 2-Naphthylamin-6-β-sulfatoethylsulfon, 1-Sulfo-2-naphthylamin-6-β-sulfatoethylsulfon, 1-Naphthylamin-4-β-sulfatoethylsulfon, 1-Sulfo-2-naphthylamin-5-β-sulfatoethylsulfon, 6-Sulfo-2-naphthylamin-8-β-sulfatoethylsulfon, 2-Amino-3-sulfonaphthalin-6,8-bis-(β-sulfatoethylsulfon), N-Methylanilin, N-Ethylanilin, N-β-Hydroxyethylanilin, N-β-Sulfatoethylanilin, 3-Aminobenzoesäure, 4-Aminobenzoesäure, Anilin, 3-Chloranilin, 4-Chloranilin.

Als chromophore Amine Y-H und/oder X-H kommen besonders Farbstoffe der folgenden Strukturtypen in Betracht: 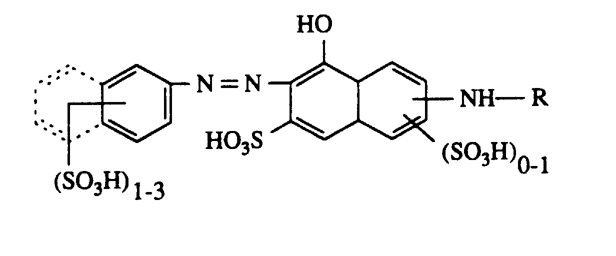 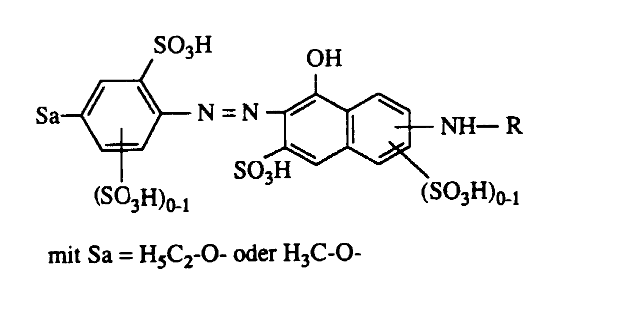 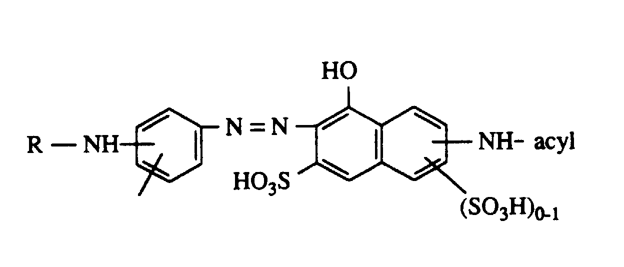 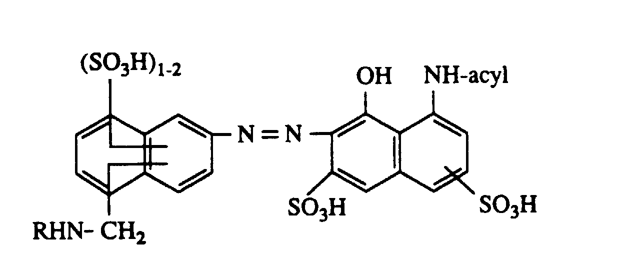 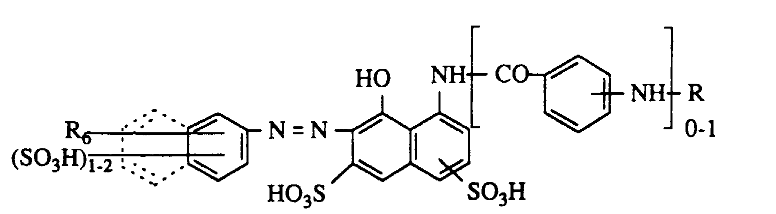 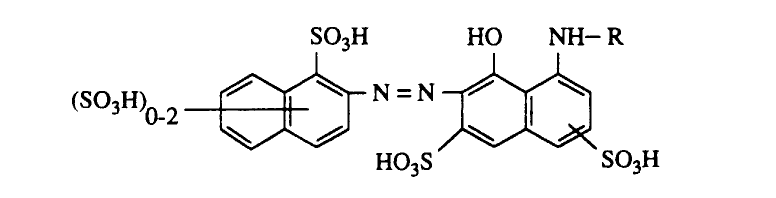 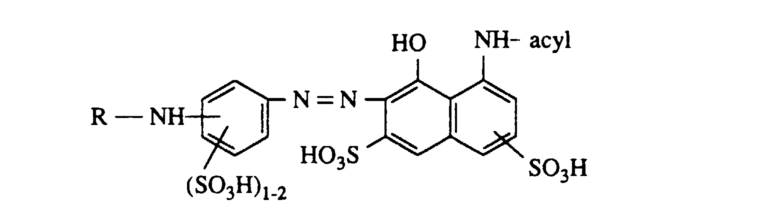 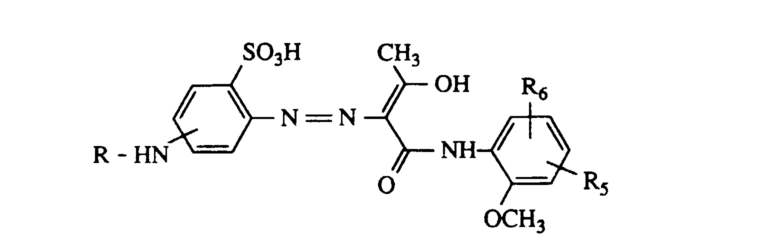 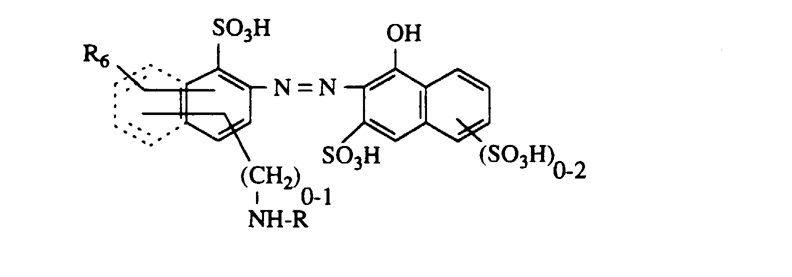 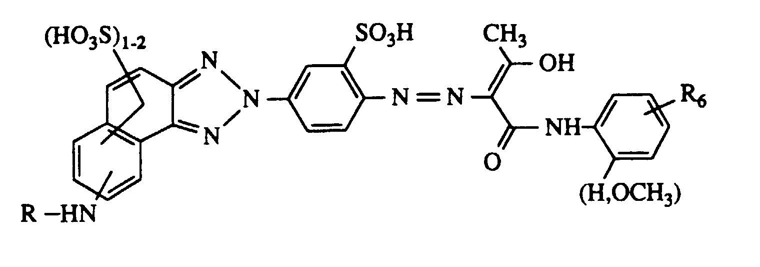 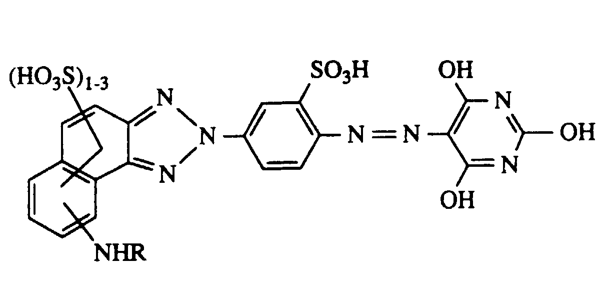 worin
acyl z.B. Acetyl oder gegebenenfalls substituiertes Benzoyl ist,
- R =: H, CH₃ oder C₂H₅,
- R₁ =: die obengenannte Bedeutung hat,
- R₅ =: H, CH₃, OCH₃ oder Cl,
- R₆ =: H, C₁-C₄-Alkyl, C₁-C₄-Alkoxy, Cl, Br, COOH, SO₃H.

Metallkomplexe von Farbstoffen der Formeln: 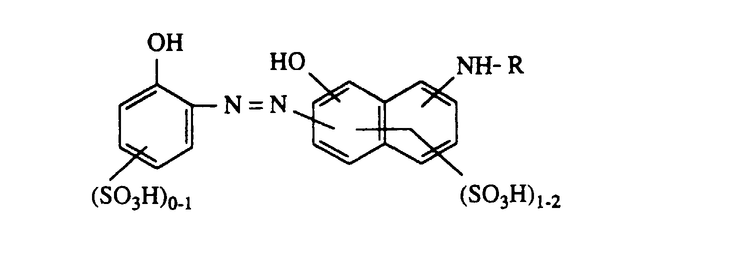 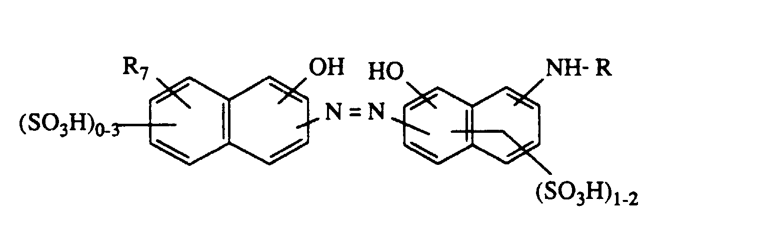 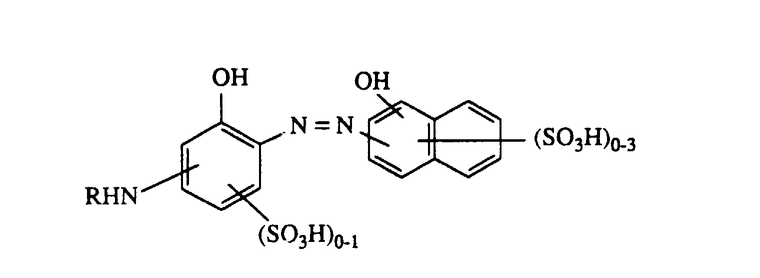 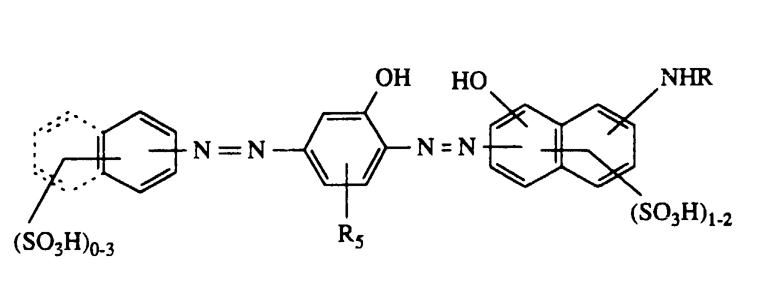 worin
- R₇ =: H, OH, NH₂, NHCOCH₃, NHCOPh, Cl, C₁-C₄-Alkoxy oder C₁-C₄-Alkyl ist.

Als Metallatom sind Cu (1:1-Komplex) oder Cr und Co (1:2-Komplex) bevorzugt. Cr- und Co-Komplexe können die Azoverbindung der oben angegebenen Formel einmal oder zweimal enthalten, d.h., sie können symmetrisch oder mit beliebigen anderen Ligandengruppen unsymmetrisch aufgebaut sein. 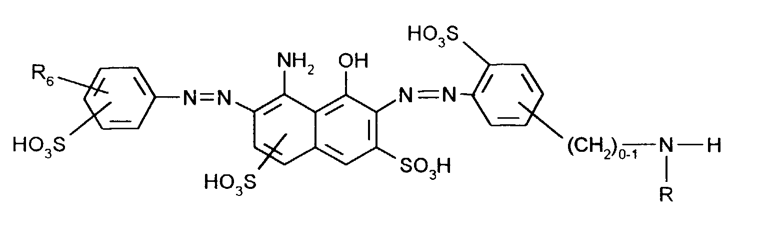 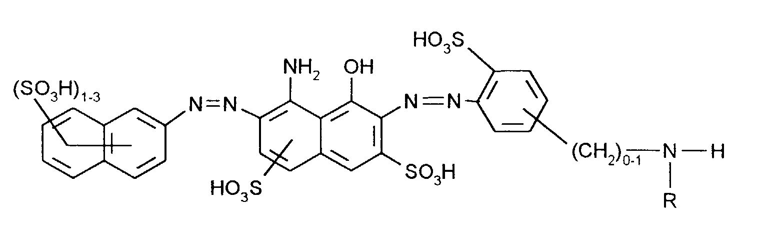 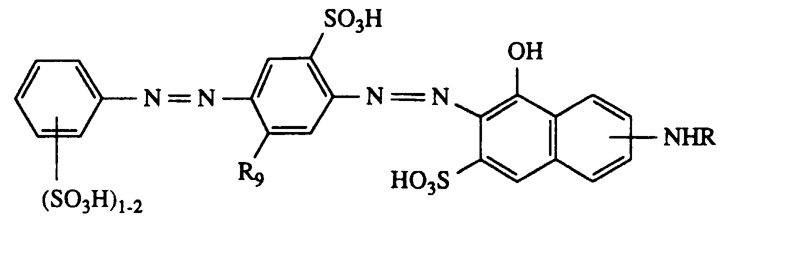 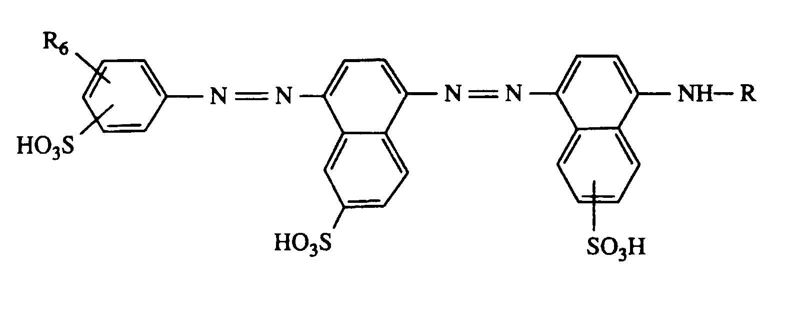 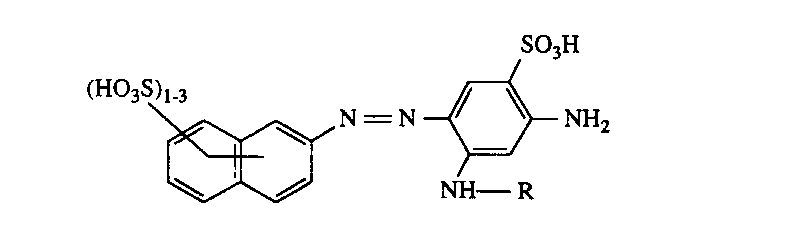 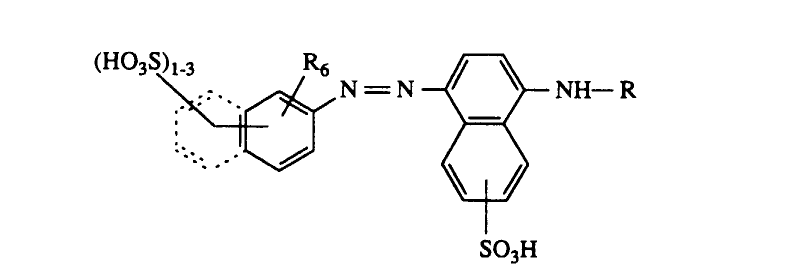 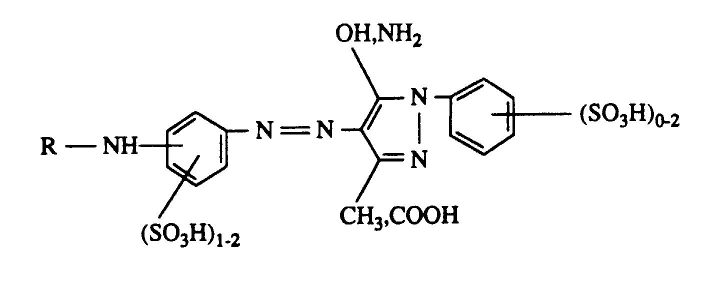 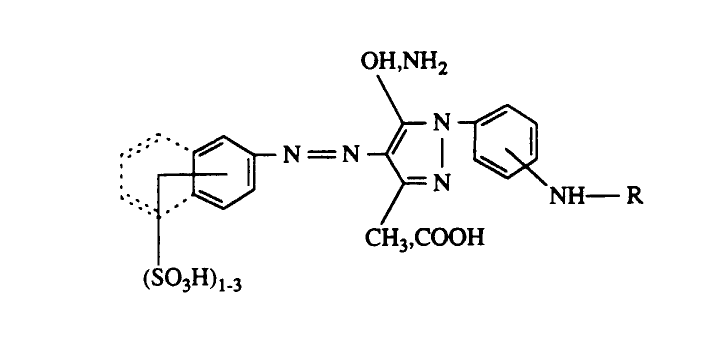 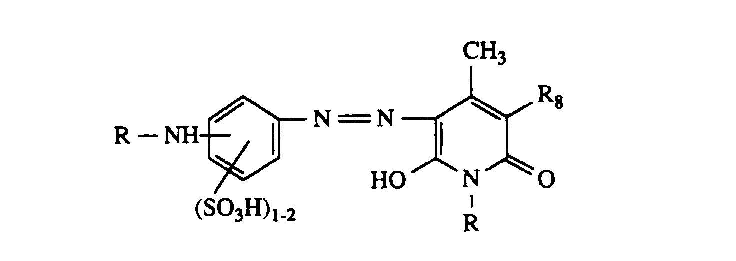 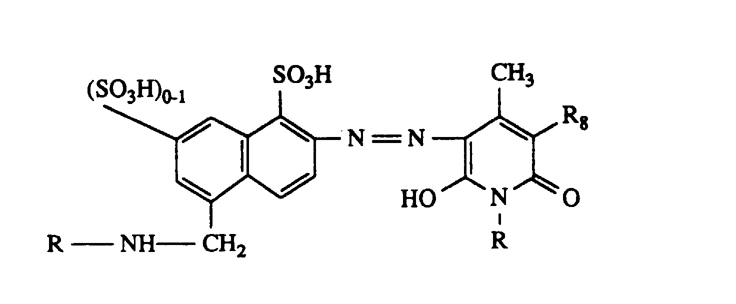 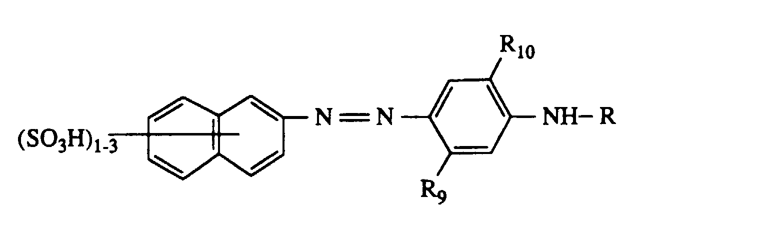 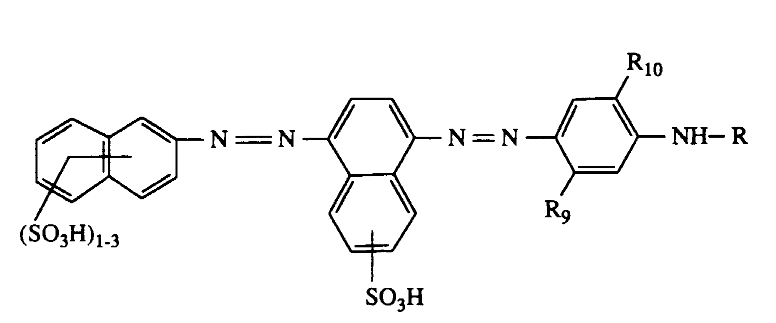 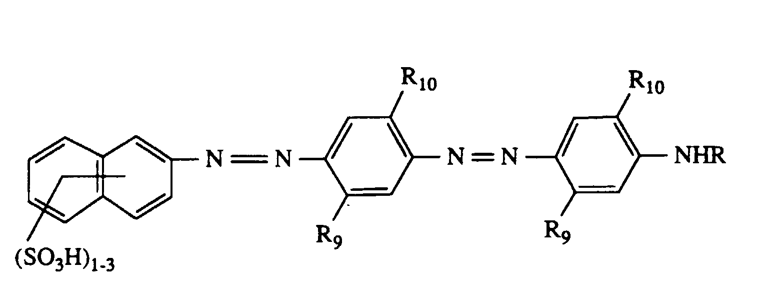 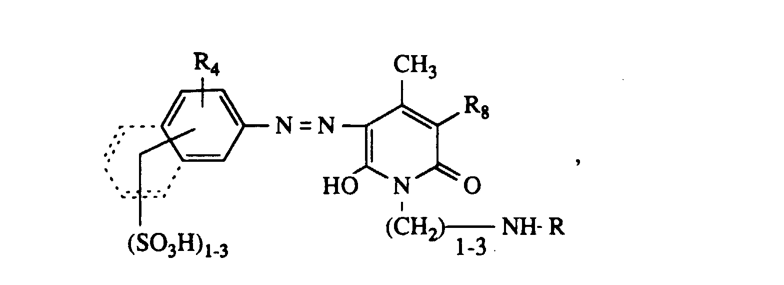 worin
- R₈ =: H, SO₃H, CH₂SO₃H, Cl, C₁-C₄-Alkylsulfonyl, CN, Carbonamid, insbesondere CONH₂,
- R₉ =: H, C₁-C₄-Alkyl, C₁-C₄-Alkoxy, Acylamino, insbesondere C₁-C₄-Alkylcarbonylamino oder Arylcarbonylamino wie gegebenenfalls substituiertes Phenylcarbonylamino, C₁-C₄-Alkylsulfonylamino, Cl, Br, Aminocarbonylamino, C₁-C₄-Alkylsulfonylamino, Arylsulfonylamino,
- R₁₀=: H, C₁-C₄-Alkyl, C₁-C₄-Alkoxy, OH, SO₃H.

Die durch Strichelungen angedeuteten kondensierten Ringe stehen für alternativ mögliche Naphthalinsysteme. 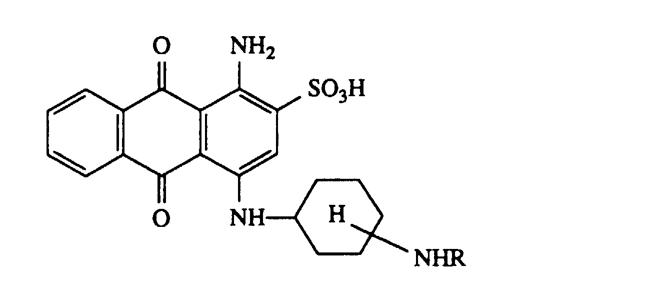 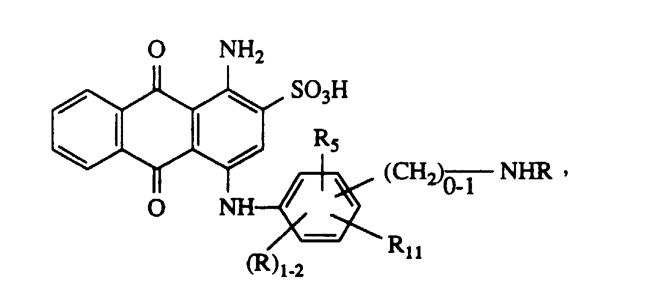 worin
- R₅ =: H, Methyl, Methoxy, Chlor
- R₁₁=: H, SO₃H und
- R =: H, Methyl, Ethyl ist. 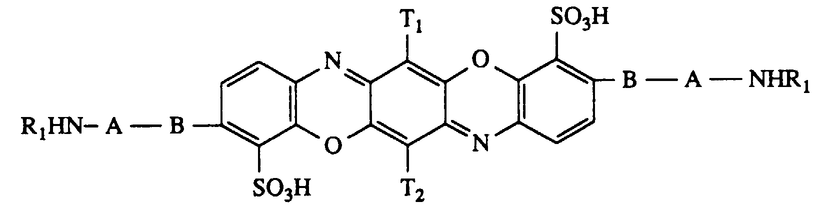
worin
- A: für ein gegebenenfalls substituiertes Phenylen oder ein gegebenenfalls substituiertes aromatisch-aliphatisches Brückenglied oder für ein gegebenenfalls durch Heteroatome wie NR, O oder S enthaltende Gruppierungen unterbrochenes, geradkettiges oder verzweigtes C₁-C₆-Alkylen, das mit OR, OSO₃H, SO₃H, COOR oder Halogen substituiert sein kann, steht.
Innerhalb eines Brückengliedes A kann NR mit NR₁ oder NR₂ auch einen heterocyclischen aliphatischen Ring bilden.
- B=: 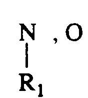 und
- T₁, T₂ =: H, Cl, Br, C₁-C₂-Alkyl, OCH₃, OC₂H₅, Acylamino, C₁-C₂-Alkoxycarbonyl ist. 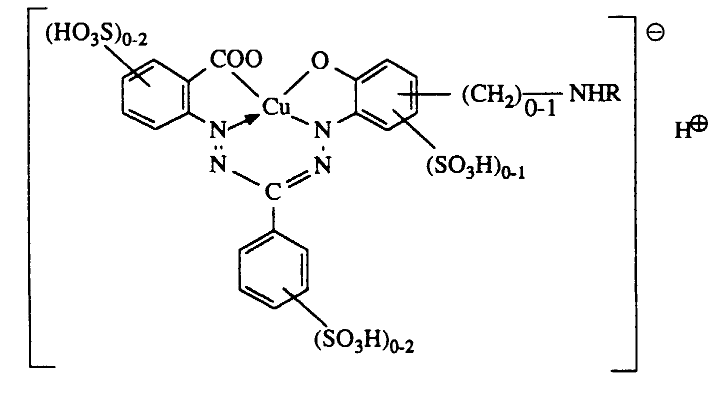 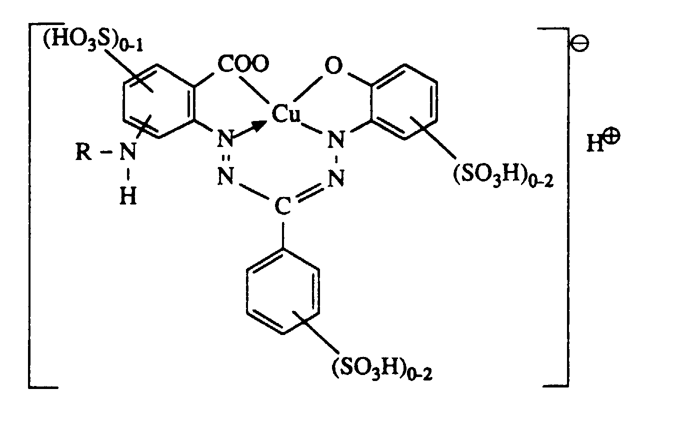 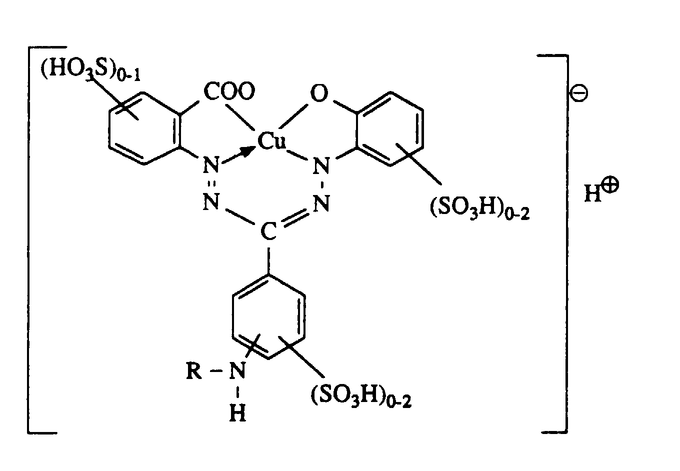 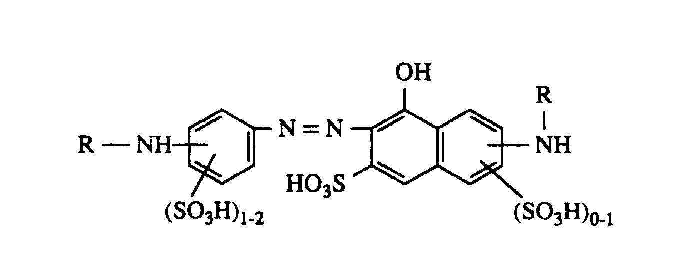 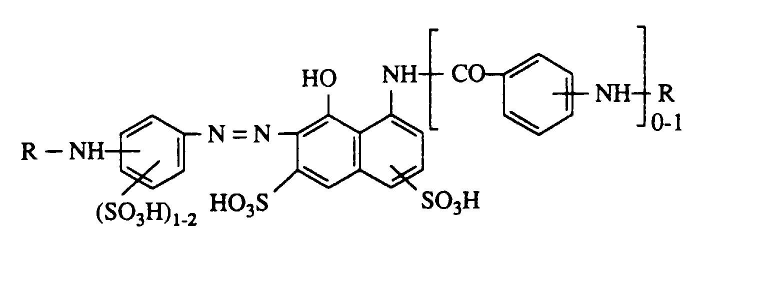 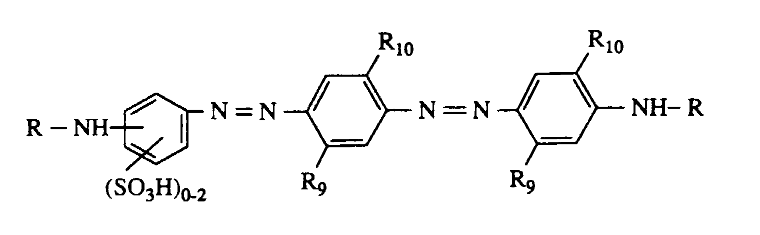 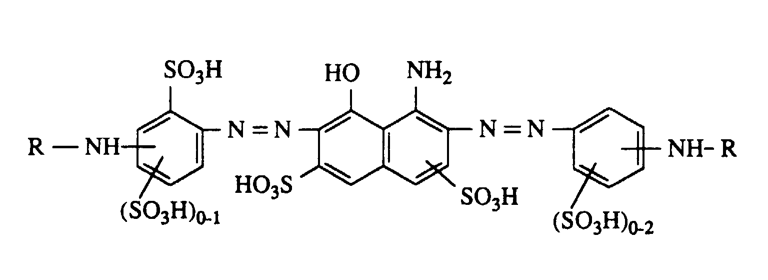 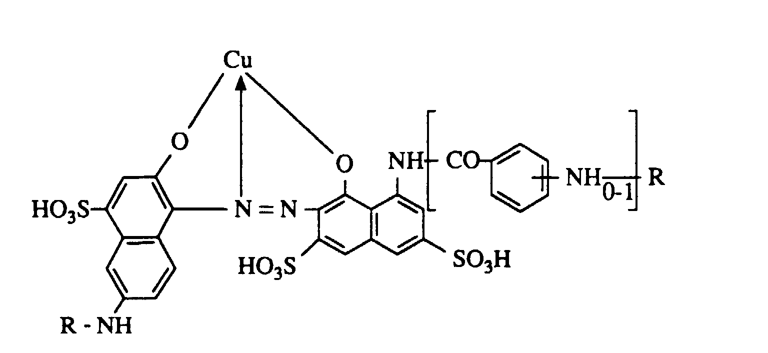 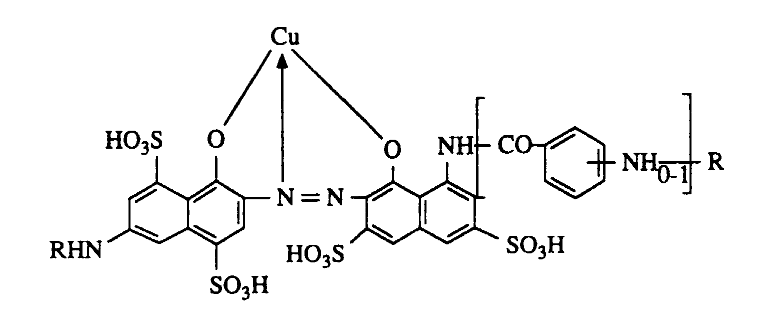 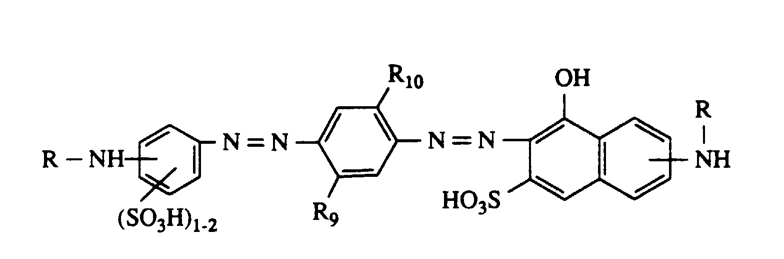 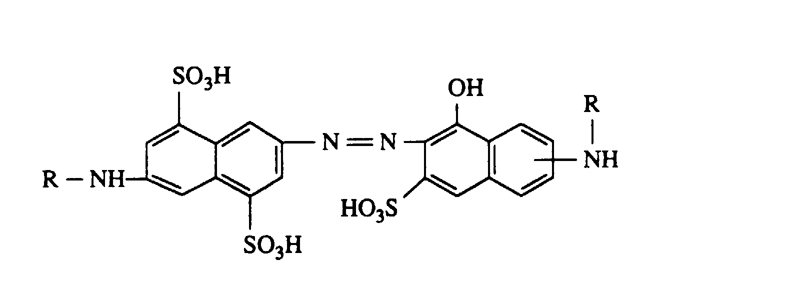 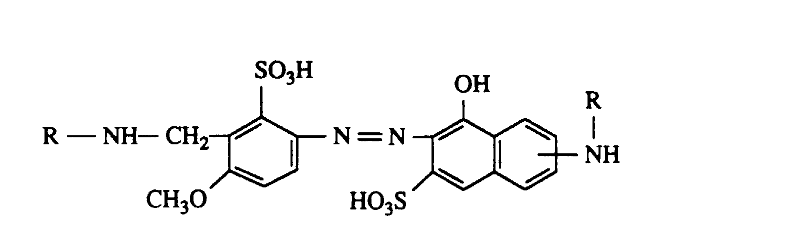 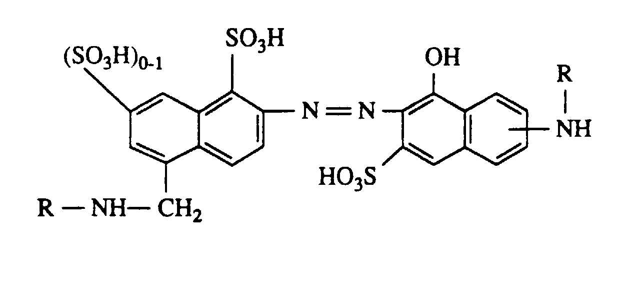 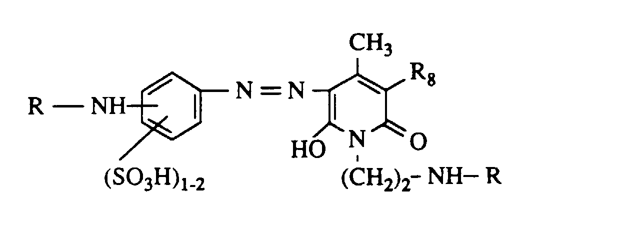 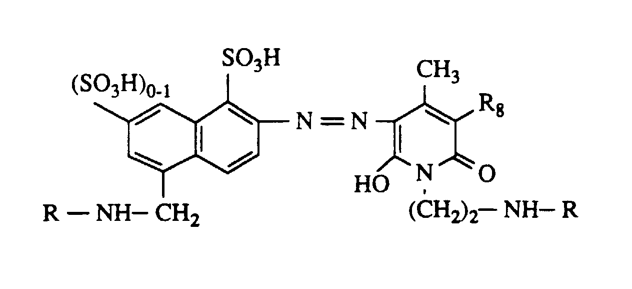 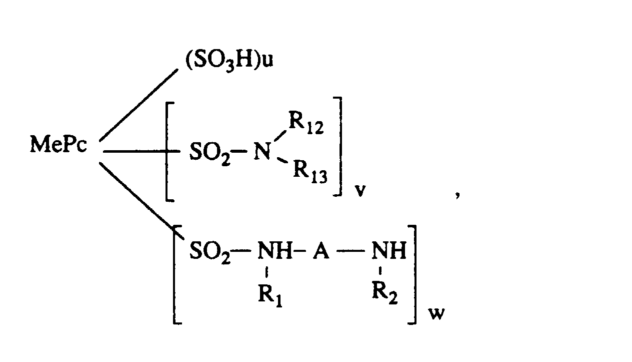
worin
- Me =: Cu,Ni ist,
- u + v + w =: 3,4-4,0 ist, mit der Maßgabe, daß u = 0,8-2,0,
- v =: 0-1,0,
- w =: 1,0-3,0 ist und

R₁ und A die oben angegebenen Bedeutungen haben,
- R₁₂ und R₁₃ =: H, C₁-C₂-Alkyl, gegebenenfalls substituiert durch OH, OSO₃H, SO₃H oder COOH ist.

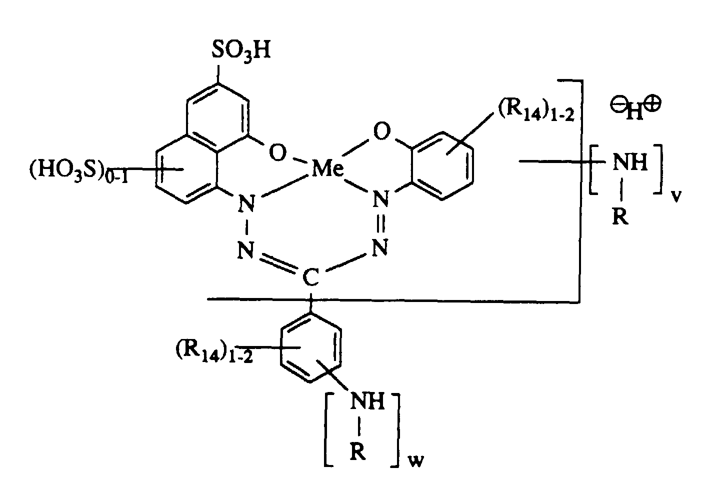

- v,w =: 0 oder 1, wobei w ungleich v,

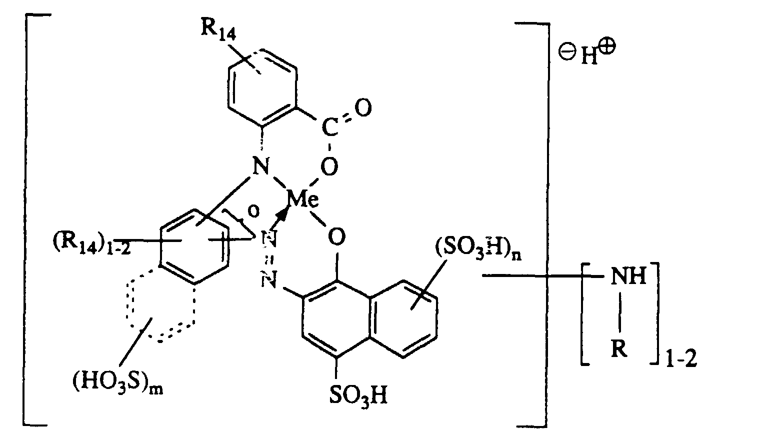

- R₁₄=: H, C₁-C₄-Alkyl, C₁-C₄-Alkoxy, OH, Halogen, COOH, NO₂, SO₃H, Sulfonamido, C₁-C₄-Alkylcarbonylamino, gegebenenfalls substituiertes Phenylcarbonylamino, C₁-C₄-Alkylsulfonylamino, gegebenenfalls substituiertes Phenylsulfonylamino,
- Me =: zweiwertiges Metallatom, vorzugsweise Fe, Cu, Zn, Co, Ni, 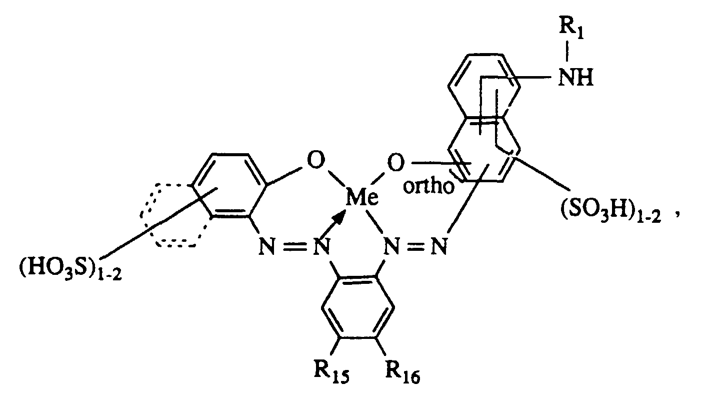
worin
- R₁₅ =: C₁-C₄-Alkyl, Halogen, insbesondere Chlor, C₁-C₄-Alkoxy, C₁-C₄-Alkylcarbonylamino, Arylcarbonylamino, Aralkylcarbonylamino,
- R₁₆=: C₁-C₄-Alkoxy, oder R₁₅ und R₁₆ einen Ring bilden und folgende Bedeutung haben:

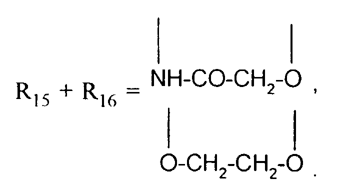

Das erfindungsgemäße Verfahren ist beispielsweise dadurch gekennzeichnet, daß die Verbindungen der Formel (I) folgender Formel entsprechen

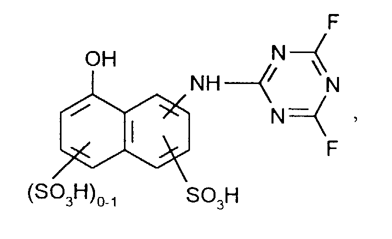

Geeignete Reaktoren sind solche, bei denen die Reaktionspartner im stöchiometrisch gewünschten Verhältnis unter hohem spezifischen Energieeintrag unter Vermeidung, zumindest aber Minimierung von Rückvermischung intensiv miteinander vermischt werden, wobei die Reaktionsbedingungen, z.B. Temperatur und pH-Wert, so gewählt werden, daß in dem Reaktor bereits ein erheblicher Umsatz erfolgt.

Geeignete Reaktoren sind beispielsweise Düsenreaktoren wie bei Zehner, P. u. Bittins, K.: Fortschr. Verf. Technik D23, 1985; S.373-393 beschrieben, bei denen die Edukte gleichzeitig und kontinuierlich mit unterschiedlichen Geschwindigkeiten in den Reaktor eingeleitet werden und durch die Differenz der Strömungsgeschwindigkeiten eine intensive Vermischung bewirkt wird, und dabei die Umsetzung bei weitgehend rückvermischungsfreier Strömung zu einem erheblichen Teil in diesem Reaktor abgeschlossen wird. Geeignet sind insbesondere auch Reaktoren von dem Typ, der in Fig. 1 der DE-A-40 16 159 angegeben ist.

Weitere geeignete Reaktoren sind Dispergiereinheiten des Typs Rotor/Stator-Mischer (O. Fuchs, Chemiker-Zeitung-Chem. Apparatur 84, Jahrgang (1960), Nr.24, S.809ff), die durch hohe Drehzahlen und kleine Reaktionsvolumen eine sehr intensive Durchmischung in kurzer Zeit erreichte.

Folgende Verfahrensvarianten unter Verwendung der erfindungsgemäßen Reaktoren sind bevorzugt:
- Eine Lösung oder Suspension von I und eine Lösung oder Suspension von Y-H werden gleichzeitig und kontinuierlich durch getrennte Zuleitungen dem Reaktor zugeführt.
   Die zur exakten Einstellung des pH-Wertes im Reaktor nötige Base wird einem oder beiden Edukten vorher zugegeben.
   Die Einstellung der Stöchiometrie erfolgt durch das Regeln der Eduktströme und die Temperierung wird durch Kühlen oder Heizen von einem oder beiden Edukten bewerkstelligt.
- Eine Mischung von I und Y-H, deren stöchiometrisches Verhältnis vorher festgelegt ist, wird als Lösung oder Suspension dem Reaktor temperiert zugeführt. Gleichzeitig und kontinuierlich wird durch eine getrennte Zuleitung die zum Erreichen des Reaktions-pH-Wertes nötige Menge Base - gegebenenfalls temperiert - in den Reaktor gefördert.
- Eine Lösung oder Suspension von I und Y-H werden dem Reaktor gleichzeitig und kontinuierlich durch getrennte Zuleitungen - gegebenenfalls temperiert - zugeführt. Die zur Umsetzung nötige Menge an Base wird durch eine dritte Zuführung gleichzeitig und kontinuierlich - gegebenenfalls tempenert - in den Reaktor gefördert.

Diese Verfahrensvarianten ermöglichen eine optimale Anpassung der Kondensationsbedingungen von I mit dem Amin Y-H bezüglich Stöchiometrie, pH-Wert und Temperatur an die Erfordernisse der Edukte, der Reaktionskinetik und des Reaktionsproduktes II.

Ein Vorteil des Verfahrens ist die weitgehende Vermeidung von Neben- und Folgeprodukten. Dies ist möglich durch die intensive Durchmischung in kurzer Zeit, die exakte Steuerung der Verfahrensparameter und die Vermeidung bzw. Minimierung der Rückvermischung.

Die Verweilzeit in dem Reaktor reicht aus, daß die Umsetzung bereits in einem erheblichen Maße erfolgt ist

Zur Beendigung der Reaktion können weitere kontinuierlich durchströmte Reaktoren wie z.B. Rotor/Stator-Systeme, Strömungsrohr evtl. mit Statikmischer und Rührwerksbehälter verwendet werden. Alternativ kann die Reaktion auch in diskontinuierlich betriebenen Rührwerksbehältern zu Ende geführt werden.

Die Zuführung der Komponenten 1, Amin Y-H und Base in den Reaktor erfolgt zum Beispiel zentral und über einen konzentrischen Ringspalt (Düsenreaktor) oder zentral und in einen oder mehrere der Rotorkränze (Rotor/Stator-Mischer).

Gemäß einer weiteren besonderen Durchführungsform des neuen Verfahrens wird die Reaktion bei Temperaturen von 0 bis 90°C, vorzugsweise 0 bis 50°C, durchgeführt.

Die Alkalimenge in der Aminlösung wird so gewählt, daß sich gegen Ende der Reaktion ein pH-Wert zwischen 4 und 11, vorzugsweise zwischen 5 und 10, einstellt.

Wird ein chromophores Amin Y-H mit einer Difluortriazinyl-Verbindung I umgesetzt, kann der erhaltene Reaktivfarbstoff isoliert oder ohne Zwischenisolierung direkt getrocknet werden.

Bei Umsetzung von I mit einem nichtchromophoren Amin kann das Reaktionsprodukt isoliert werden, es wird jedoch vorzugsweise ohne Zwischenisolierung weiterverarbeitet, z.B. zu Reaktivfarbstoffen, entweder durch nachfolgende Diazotierung und Kupplung mit einer Kupplungskomponente oder durch Umsatz mit einer Diazokomponente.

Diese Weiterverarbeitung kann diskontinuierlich oder kontinuierlich auf bekannte Art und Weise durchgeführt werden.

Nach dem erfindungsgemäßen Verfahren erhält man die Kondensationsprodukte aus der Difluortriazinyl-Verbindung I und Aminen in vielen Fällen in deutlich höherer Reinheit als nach den bisher üblichen Verfahren. Dies wirkt sich positiv auf die Qualität der aus den Kondensationsprodukten hergestellten Reaktivfarbstoffe aus, da üblicherweise die Kondensationsprodukte ohne Zwischenreinigung weiterverarbeitet werden.

### Beispiel 1

In einem Strahldüsenreaktor von 60 cm³ Volumen werden gleichzeitig und kontinuierlich über zwei getrennte Zuleitungen 40 l/h Sodalösung (200 g/l) von 20°C zentral sowie 38 l/h einer wäßrigen Lösung (pH = 4; 0°C) von 0,45 Mol/l Morpholin und 0,45 Mol/l der Verbindung (1) der Formel 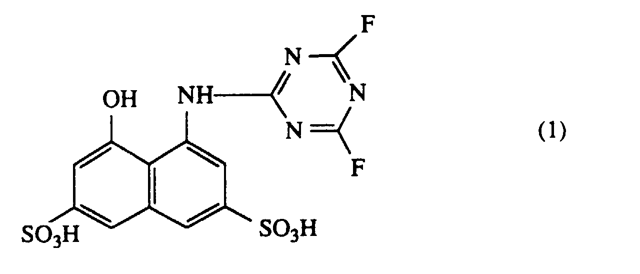 durch einen Ringspalt so eingespeist, daß die Sodalösung mit einem Druckabfall von 34 bar in die strömende wäßrige Lösung von 1 und Morpholin eintritt und die Rückvermischung im Reaktor weitgehend vermieden wird.

Das Reaktionsgemisch, das dem Reaktor mit einer Temperatur von 20 bis 21°C, pH-Wert von 8,5 bis 8,6 verläßt, enthält die Verbindung der Formel (2), 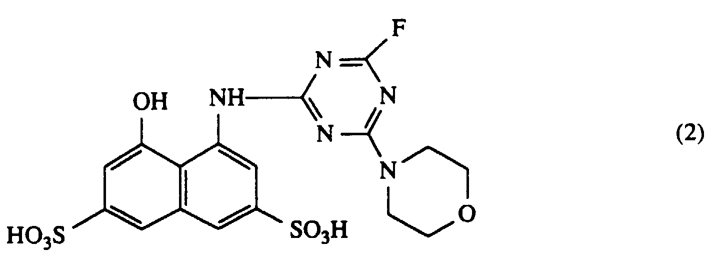 wobei der Umsatz nach dem Reaktor über 95 % beträgt und (2) in sehr hoher Reinheit erhalten wird.

(2) wird mit dem Diazoniumsalz von 2-Naphthylamin-1-sulfonsäure nach bekannten Verfahren umgesetzt. Man erhält den Reaktivfarbstoff der Formel 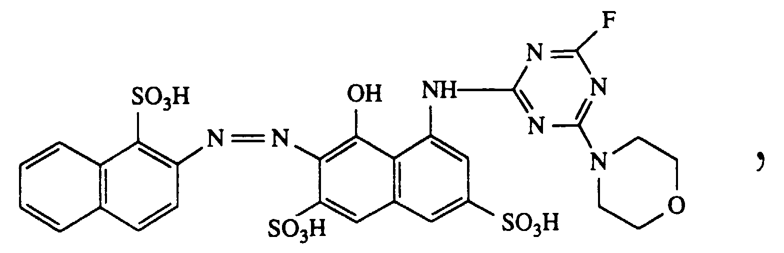 der Cellulosematerial in roten Tönen färbt.

### Beispiel 2

Verwendet man statt Morpholin analoge Mengen Anilin und speist die Lösung von (1) und Anilin zentral unter einem Druckverlust von 30 bar und die Sodalösung über den Ringspalt ein und arbeitet ansonsten analog Beispiel 1, erhält man den Farbstoff der Formel 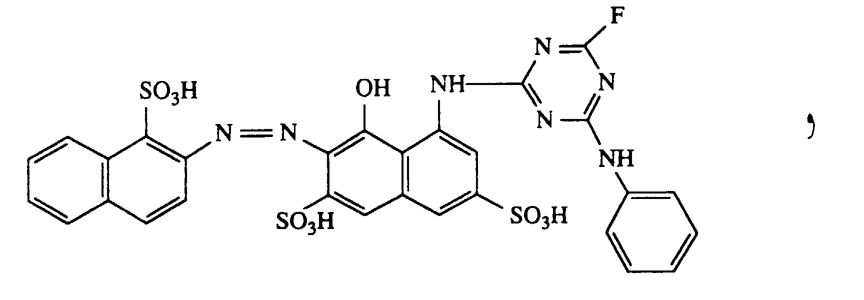 der Cellulosematerialien in klaren roten Tönen anfärbt.

### Beispiel 3

In eine Dispergiereinheit vom Typ Rotor/Stator-Mischer werden gleichzeitig und kontinuierlich zentral 50,0 kg/h einer homogenen 10°C kalten Suspension von Na₂CO₃ in Wasser (167 g/l) und auf einen der Rotorkränze 33,6 kg/h einer Lösung von 0,45 Mol/l (1) und 0,45 Mol/l Morpholin (pH = 4,0; 0°C) eingeleitet.

Das Reaktionsgemisch, das den Reaktor mit einer Temperatur von ca 15°C, pH-Wert von 9,0 bis 9,2, verläßt, enthält die Verbindung der Formel (2), 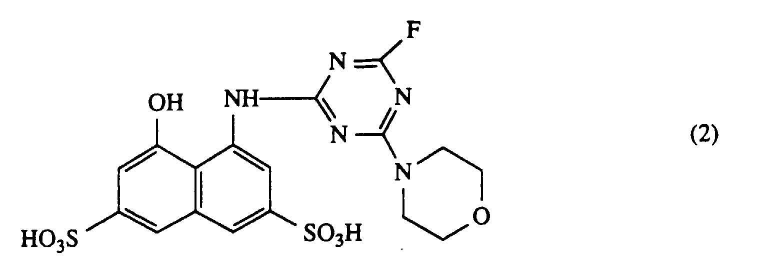 wobei der Umsatz nach dem Reaktor über 90 % beträgt und (2) in sehr hoher Reinheit erhalten wird.

(2) wird mit dem Diazoniumsalz von 2-Naphthylamin-1,5-disulfonsäure nach bekannten Verfahren umgesetzt. Man erhält den Reaktivfarbstoff der Formel 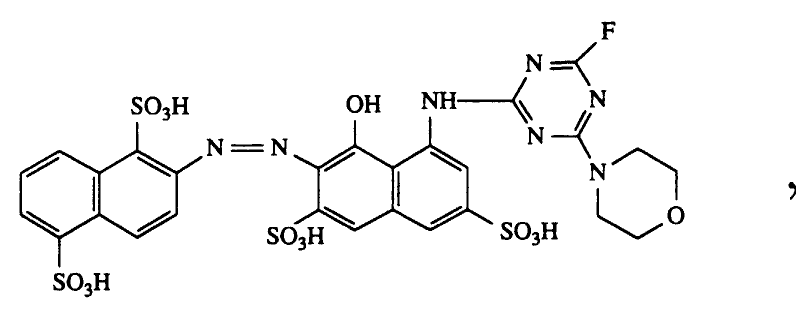 der Cellulosematerial in roten Tönen färbt.

### Beispiel 4

In eine Dispergiereinheit vom Typ Rotor/Stator-Mischer werden gleichzeitig und kontinuierlich durch drei getrennte Zuleitungen 33,6 kg/h einer Lösung von (1) (0,45 Mol/l, pH 4, 0°C) zentral und 30 l/h einer Lösung von N-Ethylanilin in Wasser 0,45 Mol/l (pH 4, 0°C) auf einen der Rotorkränze und 50 kg/h einer 10°C kalten Suspension von Na₂CO₃ in Wasser (167 g/l) auf einen anderen Rotorkranz eingespeist. Das Reaktionsgemisch, das den Reaktor verlaßt, enthält die Verbindung der Formel 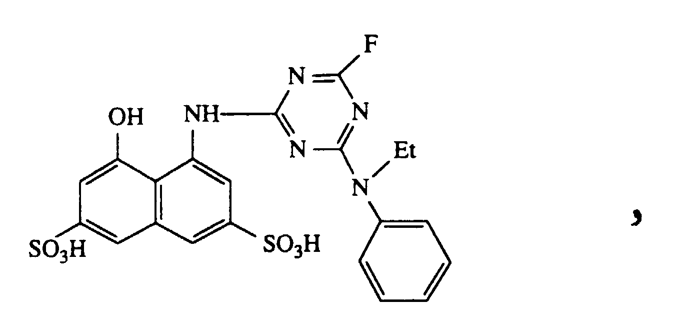 die analog Beispiel 3 zu dem Farbstoff der Formel 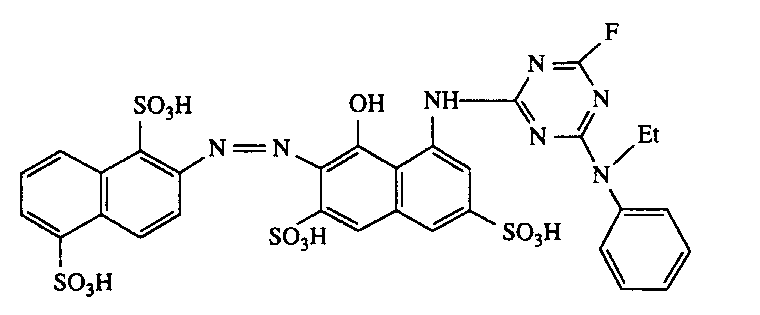 umgesetzt wird. Er färbt Baumwolle in klaren roten Tönen.

### Beispiel 5

In eine Dispergiereinheit vom Typ Rotor/Stator-Mischer werden gleichzeitig und kontinuierlich durch getrennte Zuleitungen 23 l/h einer Lösung von (3) zentral 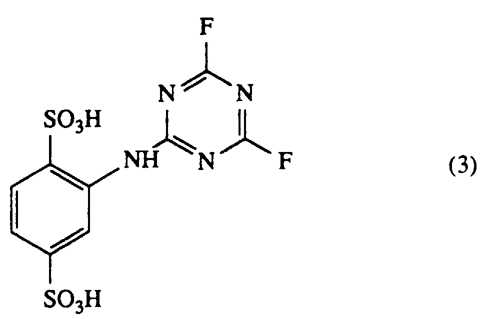 (0,29 Mol/l; pH 3; 0°C) und 38,3 kg/h einer Lösung von (4) (0,1 Mol/l, pH > 13, 10°C) auf einen der Rotorkränze geleitet.

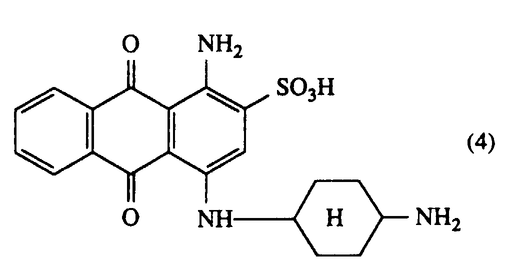

Das Reaktionsgemisch, das den Reaktor verläßt (pH 8,5; 19°C) enthält den Reaktivfarbstoff der Formel 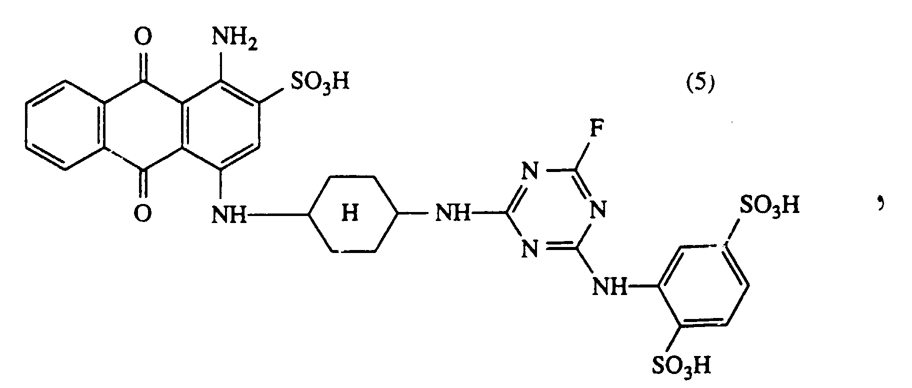 wobei der Umsatz zu (5) nach dem Reaktor mehr als 60 % beträgt. Zu Ende geführt wird die Reaktion auf einer Verweilzeitstrecke oder in einem oder mehreren Rührwerkskesseln.

## Patentansprüche

1. Verfahren zur Umsetzung von Difluortriazinyl-Verbindungen der Formel 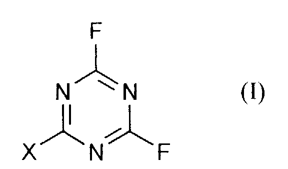 mit Aminen Y-H unter Bildung von Verbindungen der Formel II 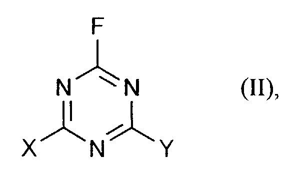 wobei :
X-H und Y-H unabhängig voneinander ein primäres oder sekundäres aliphatisches, aromatisches oder heterocyclisches Amin ist, das an einem oder beiden Resten des Stickstoff-Atomes substituiert sein kann, vorzugsweise durch OH, OSO₃H, SO₃H, COOH, Cl, OC₁-C₄-Alkyl, C₁-C₄-Alkyl, SO₂-(CH₂)₂-OSO₃H, SO₂-CH=CH₂, SO₂-(CH₂)₂-Cl, NH-Acyl, NH-C₁-C₄-Alkyl, CONH(CH₂)₂-O-(CH₂)₂-SO₂-CH=CH₂, O(CH₂)₂SO₂-CH=CH₂, O-Acyl,
wobei X-H und/oder Y-H auch chromophorhaltige Amine sein können,
dadurch gekennzeichnet, daß man die Difluortriazinyl-Verbindung vom Typ I als wäßrige oder wäßrig-organische Lösung oder als Suspension und das Amin Y-H kontinuierlich in einem Reaktor unter Vermeidung, zumindest unter Minimierung von Rückvermischung zur Umsetzung bringt.

2. Verfahren nach Anspruch 1, dadurch gekennzeichnet, daß die Verbindungen der Formel I wenigstens eine Sulfogruppe tragen.

3. Verfahren nach wenigstens einem der vorhergehenden Ansprüche, dadurch gekennzeichnet, daß die Amine X-H und/oder Y-H folgender Formel IV entsprechen 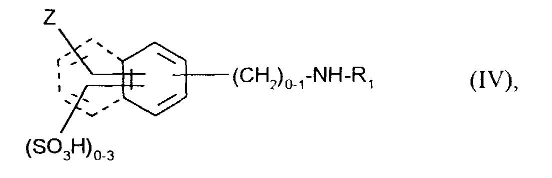 wobei:
R₁ = H, C₁-C₄-Alkyl, das durch OR, OSO₃H, SO₃H, COOR oder Halogen substituiert sein kann,
R = H, CH₃, C₂H₅,
Z = H, R, OH, Cl, OR, NH-Acyl, NHR, SO₂-CH=CH₂, SO₂-CH₂-CH₂-OSO₃H, O-Acyl, SO₂-CH₂-CH₂-Cl oder CONH(CH₂)₂O(CH₂)₂-SO₂-CH=CH₂.

4. Verfahren nach wenigstens einem der vorhergehenden Ansprüche, dadurch gekennzeichnet, daß die Verbindungen der Formel I folgender Formel entsprechen

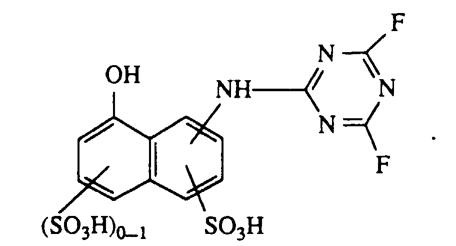

5. Verfahren gemäß wenigstens einem der vorhergehenden Ansprüche, dadurch gekennzeichnet, daß die Verbindung der Formel 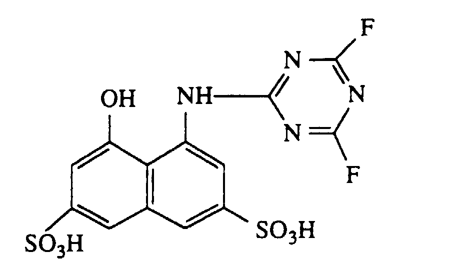 mit Morpholin umgesetzt wird.

6. Verfahren nach wenigstens einem der vorhergehenden Ansprüche, dadurch gekennzeichnet, daß die Edukte gleichzeitig und kontinuierlich mit unterschiedlicher Geschwindigkeit in einen Reaktor eingeleitet werden und durch die Differenz der Strömungsgeschwindigkeiten eine intensive Vermischung bewirkt wird, wobei die Umsetzung weitgehend rückvermischungsfrei durchgeführt wird.

7. Verfahren nach wenigstens einem der vorhergehenden Ansprüche, dadurch gekennzeichnet, daß die Edukte gleichzeitig und kontinuierlich in einen Reaktor vom Typ Rotor/Stator-Mischer geleitet werden und dort durch die hohe Drehzahl und das kleine Reaktionsvolumen in kurzer Zeit sehr intensiv durchmischt werden.

8. Verfahren nach wenigstens einem der vorhergehenden Ansprüche, dadurch gekennzeichnet, daß die Amine X-H und/oder Y-H chromophorhaltige Amine sind.

## Claims

1. Process for the reaction of difluorotriazinyl compounds of the formula I 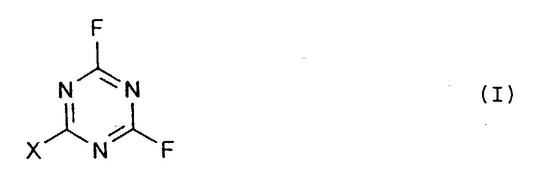 with amines Y-H with the formation of compounds of the formula II 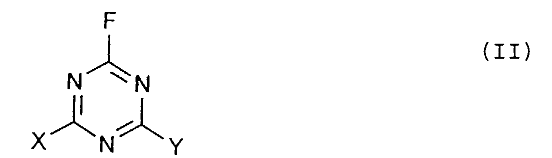 where:
X-H and Y-H are, independently of each other, a primary or secondary aliphatic, aromatic or heterocyclic amine, which can be substituted at one or both radicals of the nitrogen atom, preferably by OH, OSO₃H, SO₃H, COOH, Cl, OC₁-C₄-alkyl, C₁-C₄-alkyl, SO₂-(CH₂)₂-OSO₃H, SO₂-CH=CH₂, SO₂-(CH₂)₂-Cl, NH-acyl, NH-C₁-C₄-alkyl, CONH(CH₂)₂-O-(CH₂)₂-SO₂-CH=CH₂, O(CH₂)₂SO₂-CH=CH₂ or O-acyl,
where X-H and/or Y-H can alternatively be chromophore-containing amines,
characterised in that the difluorotriazinyl compound of type I, as an aqueous or aqueous/organic solution or as a suspension, and the amine Y-H are continuously reacted in a reactor with avoidance or at least minimisation of back-mixing.

2. Process according to Claim 1, characterised in that the compounds of the formula I bear at least one sulphonyl group.

3. Process according to at least one of the preceding claims, characterised in that the amines X-H and/or Y-H correspond to the following formula IV 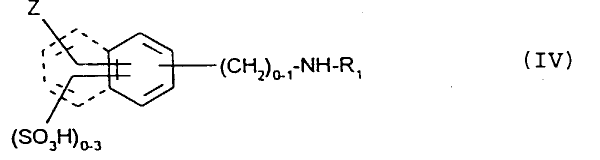 where:
R₁= H, C₁-C₄-alkyl, which can be substituted by OR, OSO₃H, SO₃H, COOR or halogen,
R = H, CH₃, C₂H₅,
Z = H, R, OH, Cl, OR, NH-acyl, NHR, SO₂-CH=CH₂, SO₂-CH₂-CH₂-OSO₃H, O-acyl, SO₂-CH₂-CH₂-Cl or CONH(CH₂)₂O (CH₂)₂-SO₂-CH=CH₂.

4. Process according to at least one of the preceding claims, characterised in that the compounds of the formula I correspond to the following formula

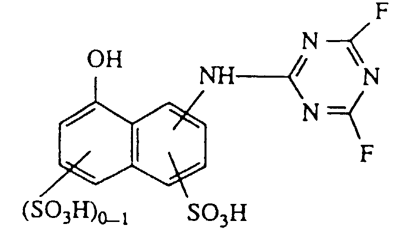

5. Process according to at least one of the preceding claims, characterised in that the compound of the formula 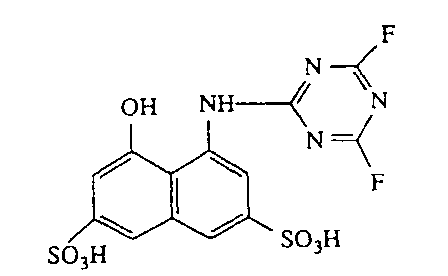 is reacted with morpholine.

6. Process according to at least one of the preceding claims, characterised in that the starting materials are passed simultaneously and continuously with different velocity into a reactor, and intensive mixing is effected by means of the difference in the flow velocities, the reaction being carried out substantially free from back-mixing.

7. Process according to at least one of the preceding claims, characterised in that the starting materials are passed simultaneously and continuously into a reactor of the rotor/stator mixer type and are highly intensively mixed there in a short period of time by means of the high rotary speed and the small reaction volume.

8. Process according to at least one of the preceding claims, characterised in that the amines X-H and/or Y-H are chromophore-containing amines.

## Revendications

1. Procédé pour la réaction de composés difluorotriazinyle de la formule (I) 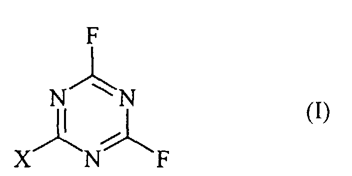 avec des amines Y-H avec formation de composés de la formule II 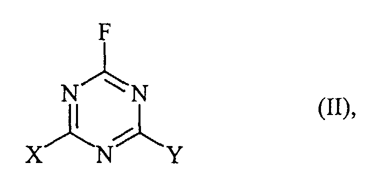 où :
X-H et Y-H sont indépendamment une amine primaire ou secondaire aliphatique, aromatique ou hétérocyclique, qui peut être substituée sur un reste ou sur les deux restes de l'atome d'azote, de préférence par OH, OSO₃H, SO₃H, COOH, Cl, O(groupe alkyle en C₁-C₄), un groupe alkyle en C₁-C₄, SO₂-(CH₂)₂-OSO₃H, SO₂-CH=CH₂, SO₂-(CH₂)₂-Cl, NH-groupe acyle, NH-groupe alkyle en C₁-C₄, CONH(CH₂)₂-O-(CH₂)₂-SO₂-CH=CH₂, O(CH₂)₂SO₂-CH=CH₂, O-groupe acyle,
X-H et/ou Y-H pouvant également être des amines contenant des chromophores, caractérisé en ce que l'on fait réagir le composé difluorotriazinyle de type I comme solution aqueuse ou aqueuse-organique ou comme suspension et l'amine Y-H en continu dans un réacteur en évitant, du moins en minimisant le remélangeage.

2. Procédé selon la revendication 1, caractérisé en ce que les composés de la formule I portent au moins un groupe sulfo.

3. Procédé selon au moins l'une quelconque des revendications précédentes, caractérisé en ce que les amines X-H et/ou Y-H correspondent à la formule IV suivante 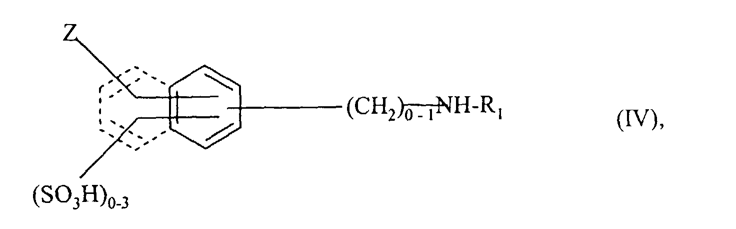 où :
R₁ = H, un groupe alkyle en C₁-C₄ qui peut être substitué par OR, OSO₃H, SO₃H, COOR ou un halogène,
R = H, CH₃, C₂H₅,
Z = H, R, OH, Cl, OR, NH-groupe acyle, NHR, SO₂-CH=CH₂, SO₂-CH₂-CH₂- OSO₃H, O-groupe acyle, SO₂-CH₂-CH₂-Cl ou CONH(CH₂)₂O(CH₂)₂-SO₂- CH=CH₂.

4. Procédé selon au moins l'une quelconque des revendications précédentes caractérisé en ce que les composés de la formule I correspondent à la formule suivante

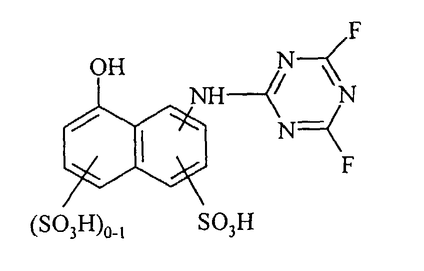

5. Procédé selon au moins l'une quelconque des revendications précédentes, caractérisé en ce que le composé de la formule 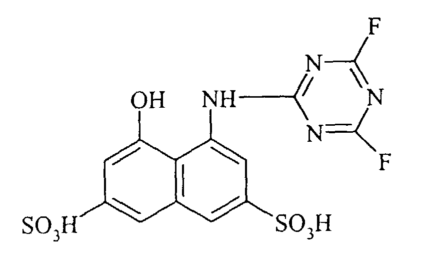 réagit avec de la morpholine.

6. Procédé selon au moins l'une quelconque des revendications précédentes caractérisé en ce que l'on introduit dans un réacteur les éduits simultanément et en continu avec une vitesse différente et en ce qu'un mélange intensif est produit par l'intermédiaire de la différence des vitesses d'écoulement, la réaction étant largement réalisée sans remélangeage.

7. Procédé selon au moins l'une quelconque des revendications précédentes, caractérisé en ce que l'on introduit dans un réacteur d'un mélangeur de type rotor/stator les éduits simultanément et en continu et qu'on les y mélange intimement de manière très intensive sur une courte durée par l'intermédiaire du nombre de rotations élevé et du faible volume de réaction.

8. Procédé selon au moins l'une quelconque des revendications précédentes, caractérisé en ce que les amines X-H et/ou Y-H sont des amines contenant des chromophores.
